# EUROPEAN PATENT SPECIFICATION

(11) **EP 2 234 595 B1**
(45) Date of publication and mention of the grant of the patent: **28.11.2012**
(21) Application number: 08859423.9
(22) Date of filing: 11.12.2008
(51) Int. Cl.: A61K 9/00, A61K 9/14, A61K 31/4015

(54) **Process for reducing the tendency of a glycopyyronium salt to aggegate during storage.**
Verfahren zur Verringerung der Neigung eines Glycopyrroniumsalzes während der Lagerung. zu aggregieren.
Procédé pour réduire la tendance d'un sel glycopyrronium pour agréger pendant le stockage.

(30) Priority: 13.12.2007 EP 07123164
(43) Date of publication of application: 06.10.2010
(73) Proprietor: Novartis AG, 4056 Basel (CH)
(72) Inventor: MUHRER, Gerhard, CH-4002 Basel (CH); RASENACK, Norbert, 79576 Weil am Rhein (DE); JUHNKE, Michael, CH-4059 Basel (CH)
(74) Representative: McLean, Craig Sutherland
(86) International application number: PCT/EP2008/067359
(87) International publication number: WO 2009/074662

(56) References cited:
- EP-A- 1 834 624
- WO-A-01/76575
- WO-A-2005/089718
- WO-A-2005/105043
- US-A1- 2002 173 536
- US-A1- 2004 037 785

## Description

This invention relates to a process for reducing the tendency of a micronised glycopyrronium salt to aggregate and/or agglomerate.

For active ingredients whose physicochemical properties make formulating difficult it is often advisable to use the most thermodynamically stable crystalline form of that active ingredient. Unfortunately some drug substances, particularly micronised dug substances, tend to aggregate on storage and/or absorb water from the atmosphere and agglomerate. This aggregation and/or agglomeration makes further processing more difficult or at least less efficient. It may even significantly affect physicochemical properties of the drug substance including stability.

Micronised glycopyrrolate, in particular, has a strong tendency to aggregate and/or agglomerate which hinders downstream drug processing, particularly the preparation of dry powder formulations for administration by inhalation.

Various processes have been proposed to process micronised drug substances to alter certain physicochemical properties of the drug substance. However some of those processes involve the use of flammable solvents that are not ideally suited to large scale commercial production. Other known solvent treatment processes, including those using water or water vapour, tend to cause local solvation processes to occur that lead to particle growth, aggregation and agglomeration.

Brodka-Pfeiffcr ct al disclosed in Drug Development and Industrial Pharmacy, Vol 29 No. 10 pages 1077-1084 (2003) that micronising salbutamol sulfate caused amorphous parts to be formed and heating the micronised substance for 5 hours at 70°C in a thermal oven failed to lead to recrystallisation and no therefore no stable product was formed.

WO 01/76575 discloses the use of glycopyrrolate in a controlled release formulation for treating respiratory diseases.

EP1834624 discloses a process for preparing fine solid drug particles involving preparing a raw liquid containing a drug, homogenising it under high pressure and removing the solvent.

WO 04/54545 discloses a process for preparing sterile aqueous suspensions of certain antibiotics or corticosteroids.

Surprisingly, it has been found that exposing a micronised drug substance to dry heat can reduce its tendency to aggregate and/or agglomerate. This even occurs when the drug substance does not contain any significantly detectable amorphous content.

Accordingly, glycopyrronium salt the present invention relates to a process for reducing the tendency of a glycopyrronium salt to aggregate and/or agglomerate during storage, the process comprising the steps of:
(a) micronising the glycopyrronium salt to give a mean particle size of less than about 10 µm; and
(b) exposing the micronised glycopyrronium salt substance to a dry environment at an elevated temperature between 40° C and 120° C for at least six hours.

The drug substance may be micronised together or "co-micronised" with one or more other drug substances and/or one or more anti-adherent agents.

Preferably the elevated temperature is between 60°C and 100°C, or suitably between 60_{°} and 90°C.

Preferably the micronised drug substance is exposed to the dry environment at the elevated temperature for 12 to 96 hours, more preferably 24 to 50 hours, especially about 24 hours.

In certain preferred embodiments when the drug substance exists in crystalline form that contains amorphous parts, the process may reduce the presence of amorphous parts in the drug substance.

The drug substance is a glycopyrronium salt, especially glycopyrrolate.

Terms used in the specification has the following meanings:
"Aggregate" as used herein means to assemble or combine together. Freshly micronised drug substances tend to take the form of a fine powder that tends to spontaneously coalesce over time to form aggregates of the drug substance. These aggregates resemble a less fine or even coarse powder.
"Agglomerate" as used herein means to form into a mass or cluster, particularly in the presence of moisture. Micronised drug substances or aggregates thereof tend on storage, particularly in the presence of moisture, to form a coarse powder, clumps or even a substantially singular mass of drug substance.
"Amorphous" as used herein described a disordered solid state, which may appear during manufacture of the drug substance (crystallization step, drying, milling) or the drug product (granulation, compression). The X-ray powder diffraction pattern of an amorphous solid exhibits no sharp peaks.
"Anti-adherent agent" as used herein means a material that reduces the cohesion between particles and prevents fine particles becoming attached to the inner surfaces of an inhaler device, or a mixture of such materials. Anti-adherent agents also include anti-friction agents or glidants, which give the powder formulation better flow properties in the inhaler. They usually lead to better dose reproducibility and higher fine particle fractions. Typical anti-adherent agents include amino acids such as leucine, phospholipids such as lecithin or fatty acid derivatives such as magnesium stearate or calcium stearate.
"Glycopyrronium salt" as used herein is intended to encompass any salt form or counterion of glycopyrronium, including but not limited to glycopyrronium bromide (glycopyrrolate), glycopyrronium chloride, or glycopyrronium iodide, as well as any and all isolated stereoisomers and mixtures or stereoisomers thereof. Derivatives of glycopyrronium salts are also encompassed. Suitable counter ions are pharmaceutically acceptable counter ions including, for example, fluoride, chloride, bromide, iodide, nitrate, sulfate, phosphate, formate, acetate, trifluoroacetate, propionate, butyrate, lactate, citrate, tartrate, malate, maleate, succinate, benzoate, p-chlorobenzoate, diphenyl-acetate or triphenylacetate, o-hydroxy-benzoate, p-hydroxybenzoate, 1-hydroxynaphthalene-2-carboxylate, 3-hydroxynaphthalene-2-carboxylate, methanesulfonate and benzene-sulfonate.
"Mean particle size" is the average diameter of particles as measured by laser light diffraction. The x90 mean particle size is the mean particle size below which 90% of particles of a sample have a lower mean particle size. The x50 mean particle size is the mean particle size below which 50% of particles of a sample have a lower mean particle size. The x10 mean particle size is the mean particle size below which 10% of particles of a sample have a lower mean particle size.

Throughout this specification and in the claims that follow, unless the context requires otherwise, the word "comprise", or variations such as "comprises" or "comprising", will be understood to imply the inclusion of a stated integer or group of integers but not the exclusion of any other integer or group of integers.

The present invention relates to a process for reducing the tendency of a glycopyrronium salt to aggregate and/or agglomerate. The process involves (a) micronising the drug substance to give a mean particle size of less than about 10 µm; and (b) exposing the micronised drug substance to a dry environment at an elevated temperature between 40°C and 120°C for at least 6 hours.

The drug substance can be any pharmacologically active ingredient although the process of the invention is especially useful for crystalline drug substances whose physicochemical properties make conventional formulation difficult, especially in the production of dry powder formulations for administration by inhalation. In general such substances have activated surfaces and sufficient chemical stability to withstand treatment at the temperature employed.

Such drug substances include anti-inflammatory, bronchodilatory, antihistamine, decongestant and anti-tussive drug substances, for example β₂-adrenoceptor agonists, antimuscarinic agents, steroids, PDE4 inhibitors, A₂ₐ agonists or calcium blockers. Preferred drug substances (including salts, polymorphs, or hydrates or solvates thereof) include antimuscarinic agents such as ipratropium bromide, tiotropium bromide, other tiotropium salts, crystalline tiotropium bromide hydrate, oxitropium bromide, aclidinium bromide, darotropium, BEA-2180, BEA-2108, CHF 4226 (Chiesi), GSK423405, GSK202423, LAS35201, SVT-40776, (R)-3-(2-Hydroxy-2,2-diphenyl-acetoxy)-1-(isoxazol-3-yl-carbamoyl-methyl)-1-azonia-bicyclo-[2.2.2]octane bromide and glycopyrronium salts; β₂-adrenoceptor agonists such as formoterol, indacaterol, albuterol (salbutamol), metaproterenol, terbutaline, salmeterol, fenoterol, procaterol, carmoterol, milveterol, BI-1744-CL, GSK159797, GSK-159802, GSK642444, PF-610355 and salts thereof; and steroids such as budesonide, beclamethasone dipropionate, fluticasone propionate, mometasone furoate, ciclesonide, GSK-685698 and 3-Methylthiophene-2-carboxylic acid (6S,9R,10S,11S,-13S,16R,17R)-9-chloro-6-fluoro-11-hydroxy-17-methoxy-carbonyl-10,13,16-trimethyl-3-oxo-6,7,8,9,10,11,12,13,14,15,16,17-dodeca-hydro-3H-cyclo-penta[a]phenanthren-17-yl ester. The solubility and other physicochemical characteristics of these substances may well affect the conditions that are required to reduce the tendency of a particular drug substance to aggregate and/or agglomerate.

In certain embodiments the drug substance may be micronised together or "co-micronised" with one or more other drug substances and/or one or more anti-adherent agents.

In the process of the present invention the drug substance is a glycopyrronium salt, especially glycopyrronium bromide or glycopyrrolate.

Glycopyrrolate, which has the chemical name 3-[(cyclopentyl-hydroxyphenylacetyl)oxy]-1,1-dimethyl-pyrrolidinium bromide, is an antimuscarinic agent that is currently administered by injection to reduce secretions during anaesthesia and or taken orally to treat gastric ulcers. However it is more recently proving to be useful in treating respiratory diseases.

Glycopyrrolate has the following chemical structure:

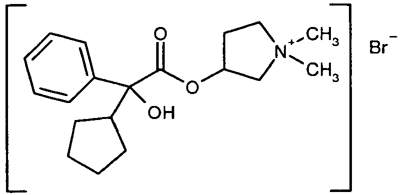

Glycopyrrolate is commercially available or can be prepared using the procedure described in United States patent US 2956062, the contents of which is incorporated herein by reference. It is preferably crystalline and contains no detectable amorphous parts.

Glycopyrrolate has two stereogenic centres and hence exists in four isomeric forms, namely (3R,2'R)-, (3S,2'R)-, (3R,2'S)- and (3S,2'S)-3-[(cyclopentyl-hydroxyphenylacetyl)oxy]-1,1-dimethylpyrrolidinium bromide, as described in United States patent specifications US 6307060 and US 6,613,795. The contents of these patent specifications are incorporated herein by reference. The present invention embraces using one or more of these isomeric forms, especially the 3S,2'R isomer, the 3R,2'R isomer or the 2S,3'R isomer, thus including single enantiomers, mixtures of diastereomers, or racemates, especially (3S,2'R/3R,2'S)-3-[(cyclopentyl-hydroxyphenylacetyl)oxy]-1,1-dimethylpyrrolidinium bromide.

The drug substance can be micronised using any suitable means to give a mean particle size of less than 10 µm, but preferably less than 5 µm. In general, drug particles of that size are suitable for administration by inhalation. Micronising, in general terms, means pulverising or otherwise reducing the drug substance using mechanical means such that at least 90% but preferably at least 95% of the resulting particulate material has a mean particle size that is less than about 10 microns in diameter. In certain embodiments the mean particle size is less than about 7 microns in diameter, and others it is less than about 5 microns in diameter.

Particles having a mean particle size greater than about 10 µm are likely to impact the walls of the throat and generally do not reach the lung. Particles having a mean particle size in the range of about 2 µm to about 5 µm will generally be deposited in the respiratory bronchioles whereas smaller particles having a mean particle size in the range of about 0.05 µm to about 2 µm are likely to be exhaled or deposited in the alveoli and to be absorbed into the bloodstream.

Micronising equipment is well known in the art and includes a variety of grinding and milling machinery, for example compressive-type mills such as mechanofusion mills, impact mills such as ball mills, homogenisers and microfluidisers, and jet mills. In a preferred embodiment crystalline glycopyrronium salt is jet milled using a Hosokawa Alpine® 100 AFG fluid bed opposed jet mill. Other suitable jet milling equipment includes Hosokawa Alpine® AFG140, AFG200, AFG280 and AFG400 jet mills, and spiral jet-mills or other milling equipment, including those operated with nitrogen, air, or other milling gases or mixtures thereof.

In some embodiments it may be suitable for the drug substance to be micronised together with an anti-adherent agent to further reduce the tendency for the drug substance to agglomerate and therefore improves the stability of the resulting drug substance. Preferably the anti-adherent agent is one or more metal stearates, one or more crystalline sugars or a mixture thereof. Especially preferred metal stearates include magnesium stearate and calcium stearate or amino acids such as leucine. Especially preferred crystalline sugars include lactose, more especially lactose monohydrate or anhydrous lactose.

The key step of the process of the present invention involves exposing the micronised drug substance to a dry environment at an elevated temperature between 40_{°} C and 120_{°} C for at least 6 hours. Such an environment is devoid or at least substantially devoid of any solvent or solvent vapour, including any organic solvents or water. In this way any no local solvation processes (due to higher water vapour affinity of activated sites on the particle surface) can occur which may otherwise lead to particle growth, aggregation and/or agglomeration.

The micronised drug substance is preferably spread thinly over trays to maximise the available surface area and those trays are placed in a chamber where they are exposed to a dry environment at an elevated temperature between 40_{°} C and 120_{°} C for at least 6 hours.

Surprisingly the process of the present invention increases the surface stability of the drug substance, especially a crystalline drug substance. Scanning electronic microscopy (SEM) photographs show exposing freshly micronised drug substances such as glycopyrrolate to increased humidity leads to particle aggregation and a smoothening of the particle surface. However freshly micronised drug substances such as glycopyrrolate that are treated according to the process of the present invention show no observable particle aggregation and the particle surfaces remain rough. This even remains the case when the same material is subsequently exposed to increased humidity, underlining the change in surface properties towards a more stable one by employing the process of the present invention.

While not wishing to be bound by theory, treating the drug substance in the dry environment appears to cause a rapid re-ordering of the surfaces of the drug substance. This avoids the local dissolution and aggregation effects that can be observed when micronised drug substances are treated with solvents or solvent vapours, particularly when the drug substance is soluble in the solvent used.

The elevated temperature and duration of treatment time will vary with the drug substance that is to be treated. Preferably the elevated temperature is between 40°C and 120°C, more preferably between 60°C and at 100°C, but especially between 60°C about 90°C. Preferably the micronised drug substance is exposed to the dry environment at the elevated temperature for 12 to 96 hours, more preferably 24 to 50 hours, especially about 24 hours.

When the drug substance is glycopyrrolate the elevated temperature is preferably between 60_{°} C and 120°C, more preferably between 70°C and 90°C, but especially about 75°C. Micronised glycopyrrolate is preferably exposed to the dry environment at the elevated temperature for 12 to 72 hours, more preferably for 20 to 60 hours, but especially about 24 hours.

In certain preferred embodiments when the drug substance exists in a crystalline form that contains amorphous parts, the process may reduce the presence of amorphous parts in the drug substance. It is known, for example from Burnett et al International Journal of Pharmaceutics 287 (2004) pp123-133, that amorphous material may be produced unintentionally through milling or compression and the presence of that material in pharmaceutical systems can directly affect the processing, storage, bioavailability and delivery properties of the material.

When the drug substance is glycopyrrolate the reduction in the tendency to aggregate and agglomerate cannot be explained by the removal of amorphous parts as glycopyrrolate contains no detectable amorphous parts. The reduction in the tendency to aggregate and agglomerate appears to be the result of reordering and a surface-energy-decrease at or near the surface of the drug substance.

Drug substances that have been treated in accordance with the process of the present invention have a reduced tendency to aggregate and/or agglomerate and thus provide a substantially stable solid bulk drug substance that facilitates further processing i.e. admixing of lactose carrier particles to give an inhalable dry powder. The treatment may also increase the ability of the drug substance to resist small to moderate increases in relative humidity by virtue that the process of the present invention has altered the surface of the drug substance particles.

The invention is illustrated by the following Example.

### Example 1

### Micronisation and solvent-free heat treatment of glycopyrrolate.

1 kg of crystalline glycopyrrolate is co-micronised with 5% magnesium stearate using a Hosokawa Alpine® 100 AFG fluid bed opposed jet mill with the following parameters: classifier speed, 17000 rpm; milling gas pressure, 4 bar. The mill is equipped with 3 nozzles of 1.9 mm diameter.

The particle size of the co-micronised glycopyrrolate and magnesium stearate is measured by laser light diffraction using a HELOS particle size analyser from Sympatec GmbH, Germany) and found to have a mean particle size of 2.5 µm.

The amorphous content of the co-micronised glycopyrrolate and magnesium stearate is measured by differential scanning calorimetry (DSC), hyper-DSC and microcalorimetry. No amorphous content is detected.

The co-micronised glycopyrrolate and magnesium stearate is spread thinly on trays and placed into a drying chamber at atmospheric conditions. The temperature in the drying chamber is raised to 70°C within one hour and maintained at that temperature for 48 hours. After that time the trays are removed from the drying chamber and left to cool.

The amorphous content of the dry heat-treated co-micronised glycopyrrolate and magnesium stearate is measured by DSC, hyper-DSC and microcalorimetry and no amorphous content is detected. Accordingly the glycopyrrolate has not undergone a phase change in the sense of a recrystallisation of amorphous parts. However surface restructuring and a decrease in surface energy is shown by inverse gas chromatography (iGC) using an inverse gas chromatograph (IGC, Surface Measurement Systems) employing the alkanes n-decane, n-nonane, n-octane, heptane, hexane and acetonitrile, and using ethanol and ethyl acetate as the probe molecules to measure dispersive surface energy and the acid-basic-ratio of surface properties.

Prior to micronisation the material shows a dispersive surface energy of 48 mJ/m² which increases during milling to approx. 70 mJ/m². After treating the material by exposure to the increased temperature, this surface energy is found to be reduced to 52 mJ/m².

A change in ka/kb-value (acid-basic ratio describing surface properties) is also noted as determined by iGC using acetonitrile, ethanol and ethylacetate as polar probe molecules: Coarse unmilled drug substance shows a ka/kb ratio of 1.0. After micronisation the ka/kb ratio of approx. 1.2, presumably as a result of new surfaces being created. After the heat treatment the ka/kb ratio is approximately. 0.9. These changes in dispersive surface energy and ka/kb ratio clearly show a change in surface properties. They indicate surface re-ordering towards an increased thermodynamic state has occurred. There is no change in particle size during this process as measured by laser light diffraction using a HELOS particle size analyser from Sympatec GmbH, Germany.

If in contrast, the material is not processed as described above, but exposed to increased temperature in combination with increased humidity in the way that the co-micronised glycopyrrolate and magnesium stearate are spread thinly on trays and placed into a chamber at atmospheric conditions. The temperature in the chamber is raised to 30°C and the relative humidity is adjusted to 75% RH within one hour and maintained at that temperature for 72 hours, dispersive surface energy is only lowered to approximately 65 mJ/m² with a ka/kb ratio of 1.4 indicating other surface properties. This underlines the benefit and potential of the described process with respect to the aim to obtain a micronised material which is thermodynamic stable and thus has a reduced surface energy.

The resulting material behaves differently from material which was not exposed to the described process. For example, if fresh micronised (or co-micronised with some force-control-agent such as magnesium stearate) is exposed to increased humidity, a particle aggregation and a smoothening of the particle surface is observed (for example seen in SEM-photographs). In contrast, if the material is exposed to the described process at increased temperature after the milling process, no particle aggregation is observed if the material is exposed to increased humidity afterwards, and also the surfaces remains rough.

## Claims

1. A process for reducing the tendency of a glycopyrronium salt to aggregate and/or agglomerate during storage, the process comprising the steps of:
(a) micronising the glycopyrronium salt to give a mean particle size of less than about 10 µm; and
(b) exposing the micronised glycopyrronium salt to a dry environment at a temperature between 40°C and 120°C for at least six hours, said environment being substantially devoid of any solvent or solvent vapour.

2. A process according to claim 1, wherein the glycopyrronium salt is co-micronised with one or more other drug substances or one or more anti-adherent agents.

3. A process according to claim 1 or 2, wherein the elevated temperature is between 60_{°} C and 100°C.

4. A process according to any preceding claim, wherein the micronised glycopyrronium salt is exposed to the dry environment for 12 to 96 hours.

5. A process according to claim 1 or 2, wherein the glycopyrronium salt is glycopyrrolate.

6. A process according to claim 5, wherein the elevated temperature is between 60_{°} C and 120_{°} C.

7. A process according to claim 5, wherein the micronised drug substance is exposed to the dry environment for 12 to 72 hours.

## Patentansprüche

1. Verfahren zur Verringerung der Neigung eines Glycopyrronium-Salzes während der Lagerung zu aggregieren und/oder zu agglomerieren, wobei das Verfahren die folgenden Stufen umfasst:
a) Mikronisieren des Glycopyrronium-Salzes zum Erhalten einer mittleren Teilchengröße von weniger als etwa 10 µm; und
b) Einwirkenlassen einer trockenen Umgebung bei einer Temperatur zwischen 40° C und 120° C während mindestens 6 Stunden auf das mikronisierte Glycopyrronium-Salz, wobei die genannte Umgebung im Wesentlichen frei von jeglichem Lösemittel oder Lösemitteldampf ist.

2. Verfahren gemäß Anspruch 1, wobei das Glycopyrronium-Salz mit einer oder mehreren weiteren Arzneimittelsubstanz(en) oder einem oder mehreren Antihaftmittel(n) co-mikronisiert wird.

3. Verfahren gemäß Anspruch 1 oder Anspruch 2, wobei die erhöhte Temperatur zwischen 60 °C und 100 °C liegt.

4. Verfahren gemäß einem der vorhergehenden Ansprüche, wobei das mikronisierte Glycopyrroniumsalz einer trockenen Umgebung für einen Zeitraum von 12 bis 96 Stunden ausgesetzt wird.

5. Verfahren nach Anspruch 1 oder 2, wobei das Glycopyrroniumsalz Glycopyrrolat ist.

6. Verfahren gemäß Anspruch 5, wobei die erhöhte Temperatur zwischen 60 °C und 120 °C liegt.

7. Verfahren gemäß Anspruch 5, wobei die mikronisierte Arzneimittelsubstanz der trockenen Umgebung für einen Zeitraum von 12 bis 72 Stunden ausgesetzt wird.

## Revendications

1. Procédé permettant de réduire la tendance d'un sel de glycopyrronium à s'agréger et/ou à s'agglomérer pendant le stockage, ledit procédé comprenant les étapes consistant à:
(a) microniser le sel de glycopyrronium pour obtenir une dimension moyenne de particules inférieure à environ 10 µm; et
(b) exposer le sel de glycopyrronium micronisé à un environnement sec à une température comprise entre 40°C et 120°C pendant au moins sis heures, ledit environnement étant substantiellement dépourvu de tout solvant ou vapeur de solvant.

2. Procédé selon la revendication 1, dans lequel le sel de glycopyrronium est co-micronisé avec une ou plusieurs autres substances médicamenteuses ou un ou plusieurs agents antiadhérants.

3. Procédé selon la revendication 1 ou 2, dans lequel la température élevée est comprise entre 60°C et 100°C.

4. Procédé selon l'une quelconque des revendications précédentes, dans lequel le sel de glycopyrronium micronisé est exposé à l'environnement sec pendant 12 à 96 heures.

5. Procédé selon la revendication 1 ou 2, dans lequel le sel de glycopyrronium est du glycopyrrolate.

6. Procédé selon la revendication 5, dans lequel la température élevée est comprise entre 60°C et 120°C.

7. Procédé selon la revendication 5, dans lequel la substance médicamenteuse micronisée est exposée à l'environnement sec pendant 12 à 72 heures.
